Europäisches Patentamt

European Patent Office

Office européen des brevets

(19)

(11) **EP 1 069 203 B1**

(12) **FASCICULE DE BREVET EUROPEEN**

(45) Date de publication et mention
de la délivrance du brevet:
**03.12.2003  Bulletin 2003/49**

(51) Int Cl.[7]: **C23C 8/24**

(21) Numéro de dépôt: **00440074.3**

(22) Date de dépôt: **15.03.2000**

(54) **Sonde pour réguler en continu l'atmosphère gazeuse dans les processus de nitruration ou de nitrocarburation dans les fours à atmosphère ou basse pression**

Sonde zur kontinuierlichen Regelung der Gasatmosphere in Nitrier- oder Nitrokarburierverfahren in Ofen mit atmospherischem oder niedrigem Druck

Probe for continuously regulating the gaseous atmosphere in nitriding or nitrocarburizing processes in atmospheric or low pressure ovens

(84) Etats contractants désignés:
**AT BE CH CY DE DK ES FI FR GB GR IE IT LI LU MC NL PT SE**

(30) Priorité:  **08.06.1999  EP 99440137**

(43) Date de publication de la demande:
**17.01.2001   Bulletin 2001/03**

(73) Titulaire: **Crevoiserat, Jean-Michel**
**2944 Bonfol (CH)**

(72) Inventeur: **Crevoiserat, Jean-Michel**
**2944 Bonfol (CH)**

(74) Mandataire: **Nithardt, Roland**
**Cabinet Nithardt & Associés S.A.,**
**14, Boulevard A. Wallach,**
**B.P. 1445**
**68071 Mulhouse Cedex (FR)**

(56) Documents cités:
**EP-A- 0 021 208        EP-A- 0 118 014**
**EP-A- 0 813 059        DE-A- 3 935 486**
**DE-A- 19 644 051        DE-C- 19 652 125**
**DE-C- 19 719 225        FR-A- 2 725 015**
**US-A- 4 417 927**

**Description**

La présente invention concerne une sonde pour réguler en continu la composition de l'atmosphère gazeuse au cours des processus de nitruration ou de nitrocarburation dans les fours à atmosphère ou basse pression.

Au cours de la nitruration ou de la nitrocarburation une couche compacte de nitrures de fer, appelée couche blanche ou couche de combinaison, se forme sur l'acier à traiter. Les atomes d'azote, de carbone et d'oxygène qui traversent cette couche diffusent vers l'intérieur et réagissent avec certains éléments d'alliage de l'acier pour former une couche dure, appelée couche de diffusion.

A l'heure actuelle, il n'existe aucun moyen pour contrôler la formation des nitrures dans l'acier et pour calculer véritablement les épaisseurs des couches. Néanmoins, il est possible de réguler les atmosphères de nitruration ou de nitrocarburation au moyen d'analyseurs, pour autant qu'il n'y ait pas de gaz additionnel comme par exemple du $CO_2$, car l'ammoniac et le $CO_2$ forment instantanément de l'urée à 62°C, ce qui empêche le prélèvement des gaz et détruit l'analyseur s'il n'est pas chauffé. Chauffer un analyseur à plus de 60°C est possible. Mais un second problème surgit avec la formation de vapeur d'eau. Il faudrait donc chauffer l'analyseur à plus de 110°C pour éviter la condensation dans les cuvettes des analyseurs.

En nitruration, on utilise principalement de l'ammoniac et de l'ammoniac craqué ou de l'azote. Dans ce cas, on peut utiliser un analyseur. En oxynitruration, avec un gaz additionnel tel que l'air, la vapeur d'eau ou le $N_2O$, la mesure en continu avec un analyseur pose les problèmes mentionnés ci-dessus.

En nitrocarburation, on observe deux tendances : l'utilisation soit de gaz additionnels tel que le $CH_4$ ou le $C_3H_8$ (sans oxygène ), soit le $CO_2$ (avec oxygène ), avec ou sans gaz endothermique. Dans ce cas, la mesure de l'atmosphère gazeuse n'est pas possible avec des analyseurs classiques.

Les appareils connus sont essentiellement destinés à réguler la composition de l'atmosphère gazeuse dans les fours de nitruration. Dans la publication US-A-4 417 927, le procédé de régulation est basé sur la mesure du débit de gaz à l'entrée et à la sortie du four. Dans la publication EP-A-0 021 208, le procédé régule l'entrée d'ammoniac et d'ammoniac craqué dans le four sur la base d'une mesure du pourcentage d'hydrogène dans le four. Ces deux procédés ne précisent pas comment les pourcentages d'hydrogène et/ou d'ammoniac sont déterminés, ce qui est fondamental pour connaître le potentiel de nitruration $K_N$.

D'autres appareils connus proposent des solutions pour calculer le potentiel de nitruration $K_N$ ou l'activité du carbone. La sonde décrite dans la publication DE-A-196 44 051 utilise une technique de mesure basée sur un électrolyte solide et celle décrite dans la publication EP-A-0 118 014 utilise une sonde à oxygène composée de dioxyde de zirconium. Ces sondes sont très limitées dans leur application et ne s'adaptent pas à des atmosphères gazeuses différentes.

La présente invention vise à pallier ces inconvénients en proposant une sonde pour réguler l'atmosphère gazeuse de nitrocarburation formées d'ammoniac et de $CO_2$, éventuellement de $N_2$ ou d'autres gaz additionnels apportant du carbone et de l'oxygène, en permettant de calculer et de réguler en continu le potentiel de nitruration $K_N$ et également l'activité du carbone, sans être influencée par la formation de composés d'ammonium (voire urée) et vapeur d'eau.

Dans ce but, l'invention concerne une sonde du genre indiqué en préambule, caractérisée en ce qu'elle comprend un module de raccordement de la sonde au four, un premier module de mesure de l'hydrogène résiduel du gaz prélevé dans le four, un module de craquage de l'ammoniac résiduel du gaz prélevé dans le four, un second module de mesure de la totalité de l'hydrogène résiduel, un module d'aspiration par effet Venturi associé à un dispositif de mesure de débit massique du gaz aspiré dans le four.

D'une manière avantageuse, la sonde peut comprendre, dans le cas d'une nitrocarburation avec un gaz oxydant, un premier et un second modules de mesure de là pression partielle d'oxygène disposés respectivement avant et après le module de craquage, ces modules de mesure de la pression partielle d'oxygène comprenant chacun une sonde Lambda.

De préférence, les différents modules sont agencés de sorte que la sonde mesure l'atmosphère gazeuse en circuit fermé, pour obtenir une mesure similaire à une mesure In Situ et ne pas provoquer une modification de la composition des gaz de l'atmosphère dans le four.

D'une manière avantageuse, le module de raccordement comprend un tube de prélèvement reliant le four au premier module de mesure de l'hydrogène résiduel et un conduit de retour reliant le module d'aspiration au four.

De préférence, le module d'aspiration du gaz prélevé dans le four comprend un conduit d'aspiration agencé pour déboucher dans le conduit de retour de manière à réaliser une aspiration des gaz à mesurer par effet Venturi au moyen d'un injecteur et d'une entrée pour l'injection d'azote, l'azote pouvant être chauffé à environ 400°C.

Le dispositif de mesure du débit massique du gaz prélevé dans le four peut comporter au moins deux capteurs de température disposés avant et après un élément de chauffage dudit gaz, ce dispositif coopérant avec un régulateur contrôlant l'injection de l'azote.

La sonde selon l'invention peut avantageusement comporter un module de calibration disposé après le module de raccordement et comportant au moins deux vannes et deux entrées de gaz de calibration disposées respectivement sur le tube de prélèvement et le conduit de retour, ces vannes pouvant être actionnées manuellement ou motorisées.

La calibration des modules de mesure de l'hydrogène résiduel peut être réalisée avec certains gaz, sans passer par le module de craquage ou à l'inverse, avec de l'ammoniac qui passera par ce module de craquage avant de passer dans les modules de mesure de l'hydrogène résiduel.

De préférence, la sonde selon l'invention comprend des moyens de chauffage et de régulation de la température des différents modules à une température supérieure à 100°C. D'une manière avantageuse, la sonde selon l'invention est reliée à un calculateur agencé pour calculer le potentiel de nitruration $K_N$ et l'activité du carbone

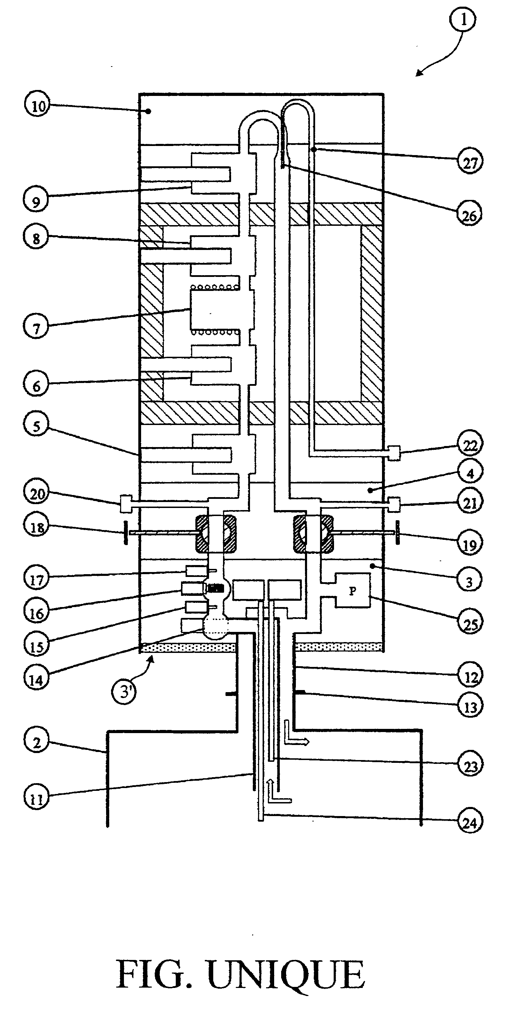

La présente invention et ses avantages apparaîtront mieux dans la description suivante d'un exemple de réalisation, en référence aux dessins annexés, dans lesquels la figure unique représente un schéma de principe de la sonde selon l'invention.

En référence à la figure unique, la sonde 1 selon l'invention est connectée à un four de nitruration ou de nitrocarburation 2 grâce à un module de raccordement 3. Elle comprend ensuite un module 4 de calibration, un premier module 5 de mesure de l'hydrogène résiduel du gaz prélevé dans le four 2, un premier module 6 de mesure de la pression partielle d'oxygène dans le four 2, un craqueur 7 avec catalyseur pour dissocier complètement l'ammoniac résiduel du gaz prélevé dans le four 2, un second module 8 de mesure de la pression partielle d'oxygène, un second module 9 de mesure de la totalité d'hydrogène résiduel, un module d'aspiration 10 du gaz prélevé dans le four 2 par effet Venturi. Ces différents modules sont agencés en circuit fermé, c'est-à-dire qu'ils sont reliés les uns aux autres et tous les modules sont chauffés à une température supérieure à 100°C.

Le module de raccordement 3 comprend un tube de prélèvement de gaz 11 en acier réfractaire, un réchauffage de canne 23 et un conduit de retour 12 sous forme d'un tube type Balzers®.Le tube de prélèvement 11 est relié à un filtre 14 puis à un dispositif de mesure du débit massique du gaz 3' comportant un premier capteur de température 15 du gaz, une bougie de chauffage 16 qui élève la température dudit gaz et un second capteur de température 17 du gaz chauffé, et agencé pour permettre la mesure du débit massique du gaz aspiré dans le four 2. La différence de température du gaz prélevé dans le four, avant et après le chauffage, est directement proportionnelle à la masse du gaz. Ce dispositif de mesure 3' est couplé à un régulateur (non représenté) qui contrôlera l'aspiration du gaz en fonction d'une consigne de débit massique fixée.

Le module de raccordement 3 comprend aussi un pressostat 25 pour mesurer la pression dans le four 2et un thermocouple 24 pour mesurer la température des gaz dans le four 2. Ces valeurs sont nécessaires au calcul du coefficient $K_N$ et de l'activité du carbone.

Le module de calibration 4 comporte, sur le tube de prélèvement 11, une vanne 18 permettant d'empêcher l'admission du gaz à analyser et, sur le conduit de retour 12, une vanne 19 permettant d'empêcher le retour des gaz dans le four 2, lors de la calibration de la sonde 1. Les vannes 18 et 19 peuvent être actionnées manuellement ou motorisées par tout moyen approprié (non représenté) pour permettre une calibration automatique de la sonde 1.

Ce module de calibration 4 comporte également, entre la vanne 18 et le premier module 5 de mesure d'hydrogène, une entrée 20 et, entre la vanne 19 et le second module 9 de mesure d'hydrogène, une entrée 21 pour l'injection des gaz de calibration. Ces gaz de calibration peuvent être composés d'azote hydrogéné à divers pourcentages, par exemple 10% $H_2$ et 90% $N_2$ pour la calibration basse et 50% $H_2$ et 50% $N_2$ pour la calibration intermédiaire et 100% NH3 qui sera complètement dissocié dans le craqueur à 800°C pour donner 75% $H_2$ et 25% $N_2$.

Le module d'aspiration 10 fonctionne en tenant compte du dispositif de mesure du débit massique 3' et permet l'aspiration du gaz dans le four 2 par effet Venturi. A cet effet, il comporte un tube d'aspiration 27 s'étendant d'une entrée 22 jusqu'à un injecteur 26 débouchant dans le conduit de retour 12. Cette entrée 22 permet l'injection d'azote N2 par l'injecteur 26 dans le module d'aspiration 10. Lors de l'injection d'azote, on crée une dépression dans le tube de prélèvement 11 ce qui a pour effet d'aspirer le gaz dans le four 2 par effet Venturi. Ce dispositif permet de supprimer l'utilisation d'une pompe rendue difficile dans un milieu à température élevée. L'azote injecté par l'entrée 22 est chauffé à environ 400°C pour éviter la formation de composés solides. L'azote injecté provient d'un régulateur électronique de pression non représenté. La pression de l'azote sur l'injecteur 26 dépend d'une boucle de régulation couplée au dispositif de mesure 3' du débit massique du gaz et qui varie en fonction d'une consigne de débit massique fixée. Entre 0,1 et 1 bar, le gaz du four 2 est aspiré proportionnellement à la pression exercée dans l'injecteur 26. Le gaz est alors mesuré par les différents modules et réinjecté avec l'azote dans le four 2.

Les modules 5 et 9 de mesure d'hydrogène sont équipés d'un capteur d'hydrogène $H_2$. En fonction de leur position dans le circuit, le premier module 5 mesure l'hydrogène résiduel du gaz prélevé dans le four 2 et le second module 9 mesure la totalité de l'hydrogène résiduel.

Le module de craquage 7 comprend un craqueur qui craque complètement l'ammoniac $NH_3$ résiduel du gaz prélevé dans le four 2. La réaction du craquage, qui transforme l'ammoniac $NH_3$ en azote $N_2$ et en hydrogène $H_2$, s'effectue à plus de 800°C. Ce module de craquage 7 comprend également un catalyseur de la réaction de craquage.

Les modules 7 et 8 de mesure de la pression partielle d'oxygène dans le gaz prélevé dans le four 2 comportent une sonde Lambda et permettent de calculer et de réguler l'activité de carbone. Ces modules 7 et 8 n'ont d'utilité que s'il y a un gaz additionnel oxydant tel que CO, $CO_2$, $N_2O$, air, etc. A l'inverse, ils n'ont pas d'utilité en cas de nitrocarburation au $CH_4$ ou $C_3H_8$.

Tous les modules de la sonde 1 sont équipés de moyens de chauffage et de régulation de la température (non représentés) permettant de maintenir une température précise à +/- 0,2°C et supérieure à 100°C pour éviter la formation de composés solides.

D'autre part, la motorisation des vannes 18 et 19 associées aux électrovannes commandant les entrées 20 et 21 permettent une calibration automatique de la sonde 1.

Cette invention, permettant de calculer l'ammoniac résiduel, est basée sur la mesure de l'hydrogène résiduel dans le four 2 au moyen du capteur $H_2$ du module 5, ensuite de craquer complètement l'ammoniac résiduel dans le module craqueur 7 et ensuite de mesurer la totalité de l'hydrogène restant au moyen d'un deuxième capteur $H_2$ du module 9. Ce principe de mesure s'applique à la nitruration et à la nitrocarburation. Le $K_N$ est calculé selon l'équation inédite suivante :

$$K_N = \frac{2 \cdot \left(x'_{H_2} - x_{H_2}\right)}{\left(3 - 2x'_{H_2}\right) \cdot x_{H_2}^{3/2}} \cdot p^{-1/2}$$

Avec

$x_{H_2}$ la fraction molaire d'hydrogène mesurée avant le craqueur
$x'_{H_2}$ la fraction molaire d'hydrogène mesurée après le craqueur
$p$ la pression dans le four

Cette invention, permettant également de calculer l'activité du carbone dans le four 2 à diverses températures de traitement en nitrocarburation avec un gaz additionnel tel que par exemple le $CO_2$ avec cette sonde, est basée sur la mesure de l'hydrogène résiduel au moyen du capteur $H_2$ du module 5 et de la mesure de la pression partielle d'oxygène au moyen d'une sonde Lambda dans le module 6 et de craquer complètement l'ammoniac résiduel dans le module 7, ensuite de mesurer la différence de la pression partielle d'oxygène obtenue au moyen d'une seconde sonde Lambda dans le module 8 et ensuite de mesurer la totalité de l'hydrogène au moyen du second capteur $H_2$ du module 9. La valeur de $K_C^B$ est calculée selon l'équation inédite suivante :

$$K_C^B = \frac{x'_{H_2} \cdot K_{H_2O} \cdot {p'_{O_2}}^{1/2} \cdot \left(3 - 2 \cdot \left(1 + K_{H_2O} \cdot {p_{O_2}}^{1/2}\right) \cdot x_{H_2}\right) - K_{H_2O} \cdot {p_{O_2}}^{1/2} \cdot x_{H_2}}{\left(K_{CO_2} \cdot {p_{O_2}}^{1/2} - K_{CO_2} {p'_{O_2}}^{1/2}\right) \cdot K_{CO_2} \cdot {p_{O_2}}^{1/2}} \cdot p$$

Avec

$x_{H_2}$ la fraction molaire d'hydrogène mesurée avant le craqueur
$x'_{H_2}$ la fraction molaire d'hydrogène mesurée après le craqueur
$p$ la pression dans le four
$p_{O_2}$ la pression partielle d'oxygène mesurée avant le craqueur
$p'_{O_2}$ la pression partielle d'oxygène mesurée après le craqueur
$K_{H_2O}$ $(T_f)$ la constante d'équilibre de la réaction $H_2 + \frac{1}{2}O_2 \leftrightarrow H_2O$
$K_{CO_2}$ $(T_f)$ la constante d'équilibre de la réaction $CO + \frac{1}{2}O_2 \leftrightarrow CO_2$
$T_f$ la température du four

En cas de non-établissement de l'équilibre du gaz à l'eau, l'équation peut être corrigée par l'introduction d'un terme empirique. La sonde 1 selon l'invention fonctionne de la façon suivante : les modules 5 et 9 de mesure d'hydrogène sont tout d'abord calibrés une fois par semaine ou une fois par jour. Si l'on souhaite une plus grande précision dans les mesures, le calibrage doit être fait avant chaque traitement. Pour cela, la vanne 18 est fermée et de l'azote est injecté par l'entrée 20 pour calibrer le "zéro" des modules 5 et 9. La vanne 18 étant fermée, on calibre le "75%" d'hydrogène du module 9 en injectant de l'ammoniac $NH_3$ par l'entrée 18. On ouvre ensuite la vanne 18 et on ferme la vanne 19 pour calibrer le "75%" d'hydrogène du module 5 en injectant de l'ammoniac $NH_3$ par l'entrée 21.

Les vannes 18 et 19 sont ensuite ouvertes et on injecte de l'azote dans le module d'aspiration 10 par l'entrée 22. Par effet d'aspiration, le gaz est alors prélevé dans le four 2 par le tube de prélèvement 11. Le gaz passe dans le module de raccordement 3 au travers du dispositif de mesure 3' du débit massique du gaz où la température du gaz est mesurée avant et après la bougie de préchauffage 16. Ce dispositif permet d'alimenter la sonde 1 avec un débit de gaz ayant une masse constante quelle que soit sa température. Ensuite le gaz passe dans le premier module 5 de mesure d'hydrogène qui détermine la quantité d'hydrogène résiduel $H_2$ dans l'atmosphère du four 2. Le gaz passe ensuite dans le module de craquage 7 dans lequel l'ammoniac résiduel est entièrement craqué (+/- 100 ppm), puis dans le second module 9 de mesure d'hydrogène qui mesure la totalité d'hydrogène résiduel.

Ces mesures sont transmises à un calculateur (non représenté) auquel est reliée la sonde 1 pour déterminer avec précision le taux d'ammoniac résiduel selon la formule:

$$\%NH_3{}^r = 2 \cdot \frac{\%H_2{}^{craqué} - \%H_2{}^{four}}{3 - \dfrac{H_2{}^{craqué}}{50}}$$

où

$NH_3{}^r$ est la quantité d'ammoniac résiduel

$H_2{}^{craqué}$ est la quantité totale d'hydrogène craqué mesurée par le module 12

$H_2{}^{four}$ est la quantité d'hydrogène résiduel dans le four mesurée par le module 10

Connaissant alors $NH_3r$, on peut calculer le potentiel de nitruration $K_N$ de façon reproductible. La détermination du $K_N$ peut se faire pour tous les types d'atmosphère de nitruration ou de nitrocarburation, le fonctionnement de la sonde 1 ne peut être influencé par la formation de composés solides.

Dans certains cas de nitrocarburation en présence de gaz additionnels, la sonde 1 comporte en plus un premier 6 et un second module 8 permettant de mesurer la pression partielle d'oxygène dans le gaz prélevé au moyen d'une sonde Lambda et de calculer l'activité de carbone

La sonde 1 permet, en mesurant deux fois le pourcentage volumique d'hydrogène, de calculer le pourcentage volumique d'ammoniac résiduel non dissocié, et donc le potentiel de nitruration $K_N$ de manière reproductible, en fonction de la pression dans le four 2, et ce pour toutes les atmosphères de nitruration et de nitrocarburation.

Cette sonde 1 permet également de calculer l'activité du carbone en fonction de la température des gaz dans le four 2, de la température du module de craquage 7, de la pression relative dans le four 2, du pourcentage volumique de l'hydrogène résiduel dans le four 2, de la pression partielle d'oxygène dans le four 2 et de la pression partielle d'oxygène et du pourcentage volumique de l'hydrogène résiduel à la sortie dudit module de craquage.

La sonde 1 fonctionnant en circuit fermé, les gaz prélevés ainsi que l'azote sont directement réinjectés dans le four 2 par le conduit de retour 12. Le gaz reste toujours à une température supérieure à 100°C évitant la formation de composés solides et la condensation.

La sonde 1 selon l'invention ne possède aucune pièce en mouvement et ne nécessite en conséquence aucun entretien.

Elle offre également l'avantage de pouvoir être adaptée au process et aux gaz utilisés en combinant les différents modules qui la composent.

## Revendications

**1.** Sonde (1) pour réguler en continu la composition de l'atmosphère gazeuse au cours des processus de nitruration ou de nitrocarburation dans les fours à atmosphère ou basse pression, **caractérisée en ce qu'**elle comprend au moins un module de raccordement (3) de la sonde (1) au four (2), un premier module (5) de mesure de l'hydrogène résiduel du gaz prélevé dans le four, un module de craquage (7) de l'ammoniac résiduel du gaz prélevé dans le four, un second module (9) de mesure de l'hydrogène mesurant la totalité de l'hydrogène résiduel, un module d'aspiration (10) par effet Venturi associé à un dispositif de mesure de débit massique (3') du gaz aspiré dans le four 2.

**2.** Sonde selon la revendication 1, **caractérisée en ce qu'**elle comprend, dans le cas d'une nitrocarburation avec un gaz oxydant, deux modules de mesure de la pression partielle d'oxygène (6, 8) disposés respectivement avant et après le module de craquage (7).

**EP 1 069 203 B1**

**3.** Sonde selon la revendication 1 ou 2, **caractérisée en ce que** les différents modules sont agencés de sorte que la sonde (1) mesure l'atmosphère du four en circuit fermé.

**4.** Sonde selon la revendication 3, **caractérisée en ce que** le module de raccordement (3) comporte un tube de prélèvement (11) reliant le four (2) au premier module (5) de mesure de l'hydrogène résiduel et un conduit de retour (12) reliant le module d'aspiration (10) au four (2).

**5.** Sonde selon la revendication 4, **caractérisée en ce que** le module d'aspiration (10) du gaz prélevé dans le four comprend un conduit d'aspiration (27) agencé pour déboucher dans le conduit de retour (12) de manière à réaliser une aspiration par effet Venturi au moyen d'un injecteur (26) et d'une entrée (22) pour l'injection d'azote.

**6.** Sonde selon la revendication 3, **caractérisée en ce que** le dispositif de mesure (3') du débit massique du gaz prélevé dans le four (2) comporte au moins deux capteurs de température (15, 17) et un élément de chauffage (16) du gaz, ce dispositif étant agencé pour coopérer avec un régulateur contrôlant l'injection de l'azote.

**7.** Sonde selon la revendication 3, **caractérisée en ce qu'**elle comporte un module de calibration (4) disposé après le module de raccordement (3) et comportant au moins deux vannes (18, 19) et deux entrées de gaz de calibration (20, 21) disposées respectivement sur le tube de prélèvement ( 11 ) et le conduit de retour (12).

**8.** Sonde selon la revendication 7, **caractérisée en ce qu'**il est prévu des unités de commande motorisées pour lesdites vannes (18, 19).

**9.** Sonde selon la revendication 2, **caractérisée en ce que** lesdits modules (6, 8) de mesure de la pression partielle d'oxygène comprennent chacun une sonde Lambda.

**10.** Sonde selon l'une quelconque des revendications précédentes, **caractérisée en ce qu'**elle comprend des moyens de chauffage et de régulation de la température des différents modules à une température précise et supérieure à 100°C.

**11.** Sonde selon la revendication 1, **caractérisée en ce qu'**elle est reliée à un calculateur agencé pour calculer le potentiel de nitruration $K_N$.

**12.** Sonde selon la revendication 2, **caractérisée en ce qu'**elle est reliée à un calculateur agencé pour calculer l'activité de carbone.


**Claims**

**1.** A probe (1) for continuously regulating the composition of the gaseous atmosphere during nitriding or nitrocarburising processes in atmospheric or low pressure furnaces, **characterised in that** it comprises at least one module (3) for connecting the probe (1) to the furnace (2), a first module (5) for measuring the residual hydrogen of the gas taken off in the furnace, a cracking module (7) for the residual ammonia of the gas taken off in the furnace, a second hydrogen measuring module (9) for measuring the entire residual hydrogen, a Venturi action suction module (10) associated with a device for measuring the mass flow rate (3') of the gas drawn into the furnace 2.

**2.** A probe according to Claim 1, **characterised in that** it comprises, in the case of nitrocarburising with an oxidising gas, two modules for measuring the partial oxygen pressure (6, 8) which are disposed respectively in front of and behind the cracking module (7).

**3.** A probe according to Claim 1 or 2, **characterised in that** the different modules are designed so that the probe (1) measures the atmosphere of the furnace in closed circuit.

**4.** A probe according to Claim 3, **characterised in that** the connection module (3) comprises a sampling tube (11) connecting the furnace (2) to the first module (5) for measuring the residual hydrogen and a return duct (12) connecting the suction module (10) to the furnace (2).

**5.** A probe according to Claim 4, **characterised in that** the suction module (10) for the gas taken off in the furnace comprises a suction pipe (27) designed to open into the return duct (12) so as to produce a Venturi action aspiration

6

by means of an injector (26) and an inlet (22) for the injection of nitrogen.

6. A probe according to Claim 3, **characterised in that** the device (3') for measuring the mass flow rate of the gas taken off in the furnace (2) comprises at least two temperature sensors (15, 17) and a heating element (16) for the gas, this device being designed to cooperate with a regulator controlling the injection of the nitrogen.

7. A probe according to Claim 3, **characterised in that** it comprises a calibration module (4) disposed after the connection module (3) and comprising at least two valves (18, 19) and two calibration gas inlets (20, 21) disposed respectively on the sampling tube (11) and the return duct (12).

8. A probe according to Claim 7, **characterised in that** it is provided with motorised control units for the said valves (18, 19).

9. A probe according to Claim 2, **characterised in that** the said modules (6, 8) for measuring the partial oxygen pressure each comprise a Lambda probe.

10. A probe according to any one of the preceding Claims, **characterised in that** it comprises means for heating and regulating the temperature of the different modules to a precise temperature of more than 100°C.

11. A probe according to Claim 1, **characterised in that** it is connected to a calculator designed to calculate the nitriding potential $K_N$.

12. A probe according to Claim 2, **characterised in that** it is connected to a calculator designed to calculate the carbon activity.

**Patentansprüche**

1. Sonde (1) zum kontinuierlichen Regeln der Zusammensetzung der Gasatmosphäre im Laufe der Nitrier- oder Nitrokarburierungsprozesse in Atmosphären- oder Niederdrucköfen, **dadurch gekennzeichnet, dass** sie wenigstens ein Modul (3) zum Anschließen der Sonde (1) an den Ofen (2), ein erstes Modul (5) zum Messen des Restwasserstoffes des aus dem Ofen entnommenen Gases, ein Modul (7) zum Kracken des Restammoniaks des aus dem Ofen entnommenen Gases, ein zweites Modul (9) zum Messen des Wasserstoffs, das die Gesamtheit des Restwasserstoffes misst, ein Modul (10) zum Ansaugen durch Venturieffekt, das einer Vorrichtung (3') zum Messen des Massenstroms des in den Ofen (2) angesaugten Gases zugeordnet ist, aufweist.

2. Sonde nach Anspruch 1, **dadurch gekennzeichnet, dass** sie im Falle einer Nitrokarburierung mit einem oxidierenden Gas zwei Module (6, 8) zum Messen des Sauerstoffteildrucks aufweist, die vor bzw. nach dem Modul (7) zum Kracken angeordnet sind.

3. Sonde nach Anspruch 1 oder 2, **dadurch gekennzeichnet, dass** die verschiedenen Module angeordnet sind, so dass die Sonde (1) die Atmosphäre des Ofens in geschlossenem Kreis misst.

4. Sonde nach Anspruch 3, **dadurch gekennzeichnet, dass** das Anschlussmodul (3) ein Entnahmerohr (11), welches den Ofen (2) mit dem ersten Modul (5) zum Messen des Restwasserstoffes verbindet, sowie eine Rückleitung (12) aufweist, welche das Ansaugmodul (10) mit dem Ofen (2) verbindet.

5. Sonde nach Anspruch 4, **dadurch gekennzeichnet, dass** das Modul (10) zum Ansaugen des au dem Ofen entnommenen Gases eine Ansaugleitung (27) aufweist, welche angeordnet ist, um in die Rückleitung (12) zu münden, derart, dass ein Ansaugen durch Venturieffekt mittels eines Injektors (26) und eines Eingangs (22) für das Einspritzen von Stickstoff realisiert wird.

6. Sonde nach Anspruch 3, **dadurch gekennzeichnet, dass** die Vorrichtung (3') zum Messen des Massenstroms des aus dem Ofen (2) entnommenen Gases wenigstens zwei Temperaturfühler (15, 17) und ein Element (16) zum Erhitzen des Gases aufweist, wobei diese Vorrichtung angeordnet ist, um mit einem Regler zusammenzuwirken, welcher das Einspritzen des Stickstoffs kontrolliert.

7. Sonde nach Anspruch 3, **dadurch gekennzeichnet, dass** sie ein Kalibrationsmodul (4) enthält, das nach dem Anschlussmodul (3) angeordnet ist und wenigstens zwei Ventile (18, 19) und zwei Kalibrationsgaseingänge (20, 21) aufweist, die an dem Entnahmerohr ( 11 ) bzw. an der Rückleitung (12) angeordnet sind.

8. Sonde nach Anspruch 7, **dadurch gekennzeichnet, dass** motorisierte Steuereinheiten für die Ventile (18, 19) vorgesehen sind.

9. Sonde nach Anspruch 2, **dadurch gekennzeichnet, dass** die Module (6, 8) zum Messen des Sauerstoffteildruckes jeweils eine Lambda-Sonde aufweisen.

10. Sonde nach irgendeinem der vorhergehenden Ansprüche, **dadurch gekennzeichnet, dass** sie Mittel zum Erwärmen und zum Regeln der Temperatur der verschiedenen Module auf eine genaue und über 100°C gelegene Temperatur enthält.

11. Sonde nach Anspruch 1, **dadurch gekennzeichnet, dass** sie mit einem Rechner verbunden ist, welcher angeordnet ist, um das Nitrierpotential $K_N$ zu berechnen.

12. Sonde nach Anspruch 2, **dadurch gekennzeichnet, dass** sie mit einem Rechner verbunden ist, welcher angeordnet ist, um die Kohlenstoffaktivität zu berechnen.

# FIG. UNIQUE